# EUROPEAN PATENT SPECIFICATION

(11) **EP 2 563 357 B1**
(45) Date of publication and mention of the grant of the patent: **17.10.2018**
(21) Application number: 11719795.4
(22) Date of filing: 29.04.2011
(51) Int. Cl.: A61K 31/192, A61P 17/02, A61P 17/10, A61K 9/00

(54) **ADAPALENE 0.3% FOR USE IN A METHOD FOR TREATING SCARS**
ADAPALENE 0,3% ZUR VERWENDUNG IN EINEM VERFAHREN ZUR BEHANDLUNG VON NARBEN
ADAPALÈNE À 0,3% POUR UTILISATION DANS UNE MÉTHODE DE TRAITEMENT DES CICATRICES

(30) Priority: 29.04.2010 US 329256 P
(43) Date of publication of application: 06.03.2013
(73) Proprietor: Galderma Research & Development, 06410 Biot (FR)
(72) Inventor: SOTO, Pascale, 06600 Antibes (FR); KERROUCHE, Nabil, 06650 Le Rouret (FR); DHUIN, Jean-Charles, 06000 Nice (FR)
(74) Representative: Starck-Loudes, Anne-Caroline
(86) International application number: PCT/EP2011/056880
(87) International publication number: WO 2011/135090

(56) References cited:
- WO-A1-2009/156679
- WO-A1-2011/014627
- WO-A1-2011/098391
- WO-A2-2004/021967
- US-A1- 2009 318 550
- US-A1- 2010 310 680
- US-B1- 6 365 623
- SHAMBAN A T ET AL: "Multimodal Treatment of Acne, Acne Scars and Pigmentation", DERMATOLOGIC CLINICS, W.B. SAUNDERS CO., LONDON, GB, vol. 27, no. 4, 1 October 2009 (2009-10-01), pages 459-471, XP008138386, ISSN: 0733-8635
- "Adapalene Gel 0.3% in the Treatment of Atrophic Acne Scars", INTERNET CITATION, 30 September 2010 (2010-09-30), pages 1-4, XP007918945, Retrieved from the Internet: URL:http://clinicaltrials.gov/ct2/show/NCT 01213199 [retrieved on 2011-06-23]
- GOODMAN GREG J ET AL: "The management of postacne scarring.", DERMATOLOGIC SURGERY : OFFICIAL PUBLICATION FOR AMERICAN SOCIETY FOR DERMATOLOGIC SURGERY [ET AL.] OCT 2007 LNKD- PUBMED:17903150, vol. 33, no. 10, October 2007 (2007-10), pages 1175-1188, XP002644931, ISSN: 1076-0512
- ALAM M ET AL: "Treatment of acne scarring.", SKIN THERAPY LETTER 2006 DEC-2007 JAN LNKD- PUBMED:17180246, vol. 11, no. 10, December 2006 (2006-12), pages 7-9, XP008138384, ISSN: 1201-5989 cited in the application
- M Patel: "Improvement in atrophic acne scars by topical adapalene (0.3% gel)", J Am Acad Dermatol, 1 May 2014 (2014-05-01), page P8622, XP055323104, Retrieved from the Internet: URL:http://www.sciencedirect.com/science/a rticle/pii/S0190962214000358/pdfft?md5=934 be8cb81d60c3dd883f2f57158be01&pid=1-s2.0-S 0190962214000358-main.pdf [retrieved on 2016-11-25]

## Description

Acne vulgaris is a chronic, inflammatory skin disease of the pilosebaceous unit, affecting approximately 80% of young adults and adolescents (Leyden JJ. et al. New understandings of the pathogenesis of acne. J Am Acad Dermatol. 1995; 32:S15-S25). Even if the treatments of acne are well known and efficient, in many cases scarring is the most unfortunate clinical outcome of acne (Layton AM, et al. A clinical evaluation of acne scarring and its incidence. Clinical and Experimental Dermatology 1994; 19:303-308). Acne scars are not uniform and there are several subtypes. Some are more hypertrophic and keloidal in appearance, while others could be more atrophic (Goodman GJ, et al. Postacne scarring - a quantitative global scarring grading system. Journal of Cosmetic Dermatology 2006; 5:48-52). The severity of the acne scars is correlated with the acne grade and also with the delay between the start of the disease and the start of an adapted treatment. Although not universal, acne scars generally occur with more inflammatory acne lesions that were not properly treated (Layton AM, et al. Scarred for life? Dermatology 1997; 195 (suppl 1):15-21). Once established, acne scars are believed not to be treatable medically but invasive procedures could offer some improvement (Jemec GBE, Jemec B. Acne: Treatment of Scars. Clinics in Dermatology 2004; 22:434-438)
Loss of dermal matrix has to be a contributing factor in atrophic acne scars (Kang S, et al. Inflammation and extracellular matrix degradation mediated by activated transcription factors NFkB and AP-I in inflammatory acne lesions in vivo. Am J Pathol 2005;166:1691-1699). This loss of dermal matrix consists in degradation of collagen produced by intracellular pathway activated by inflammation during the acne lesion formation. This leads to transcription factor activation coding for matrix-degrading metalloproteinases (MMPs). MMPs degrade extracellular matrix molecules during physiological and pathological tissue remodeling. Three MMPs (MMP-1 [collagenase], MMP-3 [stromelysin] that can initiate collagen degradation and MMP-9 [gelatinase] that can further degrade collagen fragments) have been shown to be increased in inflammatory acne lesion in vivo (Papakonstantinou E, et al. Matrix Metalloproteinases of epithelial origin in facial sebum of patients with acne and their regulations by isotretinoin. J Invest Dermatol. 2005; 125:673-684. and also Trivedi NR et al. Gene Array Expression Profiling in Acne Lesions. J of Invest Dermatol. 2006;126(5):10711079).
Another skin condition characterized by loss of dermal matrix is photoaging (Kang S, Fisher GJ, Voorhees JJ. Photoaging and topical tretinoin: therapy, pathogenesis, and prevention. Arch Dermatol. 1997; 133:1280-1284). It has been demonstrated that UV radiation activates transcription factor that lead to collagen degradation (Fisher GJ, Datta S, Wang ZQ, et al. c-Jun-dependent inhibition of cutaneous procollagen transcription following ultraviolet irradiation is reversed by all-trans retinoic acid. J Clin Invest 2000; 106:663-670). Involved proteins are the same as for inflammatory acne lesions (collagenase, stromelysin respectively MMP-1, 3 and gelatinase, MMP-9) (Kang S, Voorhees JJ. Photoaging therapy with topical tretinoin: an evidence-based analysis. JAm Acad Dermatol. 1998; 39:S55-61).

Over the past decades, significant evidence has been accumulated that topical retinoids can stimulate dermal fibroblasts to produce more procollagen in photoaged skin. Type I and type III procollagen are reduced in photodamage skin but it has been demonstrated that isotretinoin, either oral or topical, protects against UV-induced loss of procollagen, leading to MMP-1, 3 and MMP-9 decrease in relation to the duration of the treatment (Kang S, Voorhees JJ. Photoaging therapy with topical tretinoin: an evidence-based analysis. J Am Acad Dermatol. 1998;39S55-61).

This increase in collagen production is clinically appreciated as effacement of wrinkles. After 24 weeks of treatment with topical retinoid, an increase of the epidermal thickness has been observed (Griffiths CEM, et al. Two concentrations of topical tretinoin (retinoic acid) cause similar improvement of photoaging but different degrees of irritation: a double-blind, vehicle controlled comparison of tretinoin 0.1% and 0.025% creams. Arch Dermatol. 1995; 131:1037-1044).

Adapalene Gel 0.3% (Differin®) is approved in USA for the treatment of acne and has been found to be as effective as other retinoids. It was shown to have a favorable tolerability profile in comparison with other retinoids. Adapalene Gel 0.1% and 0.3% formulations have been demonstrated to treat actinic keratoses, and solar lentigines and improve some of the effects of photodamage such as fine and coarse wrinkles, mottled hyperpigmentation, and sallow complexion (Kang S, Goldfarb MT, Weiss JS, et al. Assessment of Adapalene gel for the treatment of actinic keratoses and lentigines: A randomized trial. J Am Acad Dermatol. 2003; 49:1; 83-90).

As atrophic acne scars may cause serious physical and emotional scarring and can significantly impact the quality of life (Koo JY, Smith LL. Psychologic aspects of acne. Pediatr Dermatol. 1991, 8(3):185-188) the aim of the present invention is to provide a method for treating scars and particularly acne scars.
Scars are marks created during the healing of damage to the skin or tissues. A scar is permanent and cannot be completely removed. However, treatment can alter a scar's appearance. These procedures range from the application of over-the-counter ointment to surgery. Scar treatment should start after an injury because wound care affects scarring. The wound should be cleaned and covered. Picking at the scab breaks the collagen and allows germs to enter the wound. Time also helps with healing. Scars become smaller, and the color fades.
However, additional treatment is required for some scars. While some procedures are more effective for keloids and hypertrophic scars, the procedure for acne scars is based on the type of scarring.
Therefore, there is a need of an effective treatment treating scars and/or preventing their setting up or appearance.

Hence, the first object herein described is a method for treating at least one scar where an effective amount of adapalene is administered to the patient in need thereof.
Preferably Adapalene is in a quantity of 0.3% of weight relative to the total weight of the composition.
According to said method the effective amount of adapalene is in a composition in the form of an aqueous gel. The scars treated according to said method is preferentially acne scar. Specifically, acne scar is selected from atrophic scar and hypertrophic scare. More specifically acne scar is selected from ice pick, boxcar, rolling, bridges and tunnels, gross atrophy, dystrophic and keloid scars.
According to one specific aspect, the effective amount of adapalene is topically administrated and more specifically is applied onto scarred skin. In a preferred aspect it is topically administered, preferentially onto the skin at 0.3% of weight relative to the total weight of the composition.
In another aspect, the invention concerns the use of a composition comprising adapalene for non-therapeutic treatment of acne scar according to the appended claims.
In another aspect, the description concerns the use of Adapalene at 0.3% for the preparation of a medicament which is intended for the treatment of scars and preferably acne scars. Accordingly said use is characterized in that the medicament is in the form of a composition suitable for topical application. Preferentially the medicament is in the form of a gel, emulsion, lotion. In a preferred aspect, the medicament is in the form of a composition comprising aqueous gel having the following composition, as percentage by weight with respect to the total weight:

| | |
|---|---|
| Adapalene | 0.3% |
| Carbomer | 1.1% |
| Disodium edentate | 0.1% |
| Methyl paraben | 0.2% |
| Poloxamer | 0.2% |
| Propylene glycol | 4.0 % |
| Sodium hydroxide amount required to obtain pH 5.0 +/- 0.3 and | |
| Purified water | q.s. 100% |

Thus, another aspect herein described relates to a composition comprising adapalene or its salts at a concentration of 0,3% for the treatment of the scars induced by acne, said composition being preferentially in the form of an aqueous gel.

Therefore a method herein described is a method for treating at least one scar where an effective amount of adapalene 0.3% is administrated to the patient in need thereof. Preferentially, the at least one scar is an acne scar.
The scope of the invention is defined by the claims. Any subject-matter falling outside the scope of the claims is provided for information purposes only. Any references in the description to methods of treatment refer to the compositions and medicaments of the present invention for use in a method for treatment of the human (animal) body therapy. Even with the most careful and conscientious treatment, acne scars may still occur. But not all scars are created equal. Generally, acne scars fall under two categories: those caused by a loss of tissue (atrophic), and those caused by an excess of tissue (hypertrophic). Within these categories, acne scars fall into one of four types: ice, pick, boxcar, rolling and keloid scars.
Prior art provides definition of acne scars (Alam M, Dover JS. "Treatment of Acne Scarring." Skin Therapy Letter. Dec 2006-Jan 2007; 11(10); Goodman GJ, Baron JA. "The management of post-acne scarring." Dematologic Surgery. Oct 2007; 33(10)/1175-1188.; Jacob CI, Dover JS, Kaminer MS. "Acne Scarring: a classification system and review of treatment options." Journal of the American Academy of Dermatology. 2001;45(1): 109-117.):
- Ice pick scars are deep, very narrow scars that extend into the dermis. The skin looks as if it has been pierced by an ice pick or sharp instrument. Ice pick scars seems to make a small deep "hole" in the skin. Some may look like a large, open pore. Ice pick scars develop after an infection from a cyst or other deep inflamed blemish works its way to the surface. Skin tissue is destroyed, leaving a long column-like scar. Ice pick scars can commonly be treated with punch excision or punch grafting.
- Boxar scars are depressed acne scars, round or oval in shape with steeply and angled sides. They are similar in appearance to chickenpox scars. Boxcar scars occur when an inflamed acne lesion destroy tissue, leaving a sunken area on the skin. Boxcar scars may be mild and superficial, or deeper and more severe.
When an inflammatory breakout destroys collagen, tissue; is lost. The skin over this area is left without support, and a depressed area is created. Boxcar scars may be superficial to severe, depending on the amount of tissue lost. The common treatments for boxcar scars include punch excision or elevation, dermal fillers, and laser resurfacing.
- Rolling scars arise when fibrous bands of tissue develop between the skin and the subcutaneous tissue below. These bands pull the epidermis, binding it to deeper structures of the skin. It is this pulling of the epidermis from within that creates the rolling appearance of the skin. This type of scarring causes rolling or "wave-like" undulations across otherwise normal appearing skin. Rolling scars are best treated with subcision.
- A hypertrophic scar looks like a raised, firm mass of tissue. These types of scars often grow larger than the original wound. Hypertrophic scars caused by acne are most often found on the torso, especially in men. Unlike ice pick or boxcar scars, hypertrophic scars are not caused by a loss of tissue. Rather, they develop because of an overproduction of collagen. The common treatments are selected from: Steroid (cortisone) creams, tapes, or injection to help shrink and flatten the scar. Interferon injections are also used to soften scar tissue.

A scar is a manifestation of the skin's healing process. After skin or tissue is wounded, the body releases collagen to mend the damage. Scarring is the natural process of repairing an open wound, injury, surgical incision, or other conditions like acne. Causes of scars include cuts, sores, surgery, and burns. Severe acne, varicella and chicken pox may also scar skin. Therefore, the present description encompassed other types of scar like those mentioned above.

In a preferred embodiment, the effective amount of adapalene 0.3% is topically administrated and particularly is applied on scarred skin and preferentially in a composition comprising adapalene or its salts at a concentration of 0,3%.
In the context of the invention, it is meant by « effective amount » the quantity or the concentration of Adapalene in the composition to provide the desired effect.
Hence, any formulation of adapalene at 0.3% is usable in the context of the invention such as a gel, an emulsion or a solution.

Another aspect of the description regards the use of Adapalene at 0.3% for the preparation of a medicament which is intended for the treatment of scars and preferentially acne scars like those described above.
Advantageously, the medicament according to the present invention is intended for topical application.
The medicament according to the present invention also comprises a physiologically acceptable medium, that is to say a medium which is compatible with the skin, including the scalp, mucous membranes, hair, body hairs and/or eyes, and can constitute a dermatological composition.

The compound 6-[3-(1-Adamantyl)-4-methoxyphenyl]-2-naphthoic acid (referred to hereinbelow as adapalene) is a naphthoic acid derivative with retinoid and antiinflammatory properties as well as its salts.
The term "adapalene salts" means the salts formed with a pharmaceutically acceptable base, especially mineral bases such as sodium hydroxide, potassium hydroxide and ammonia or organic bases such as lysine, arginine or N-methylglucamine. The term "adapalene salts" also means the salts formed with fatty amines such as dioctylamine and stearylamine.

Adapalene is sold under the brand name Differin® at a weight concentration of 0.1 %, in the form of an "alcoholic lotion" solution, an aqueous gel and a cream. These compositions are intended for treating acne. Patent application FR 2 837 101 describes adapalene compositions at a weight concentration of 0.3%, for treating acne.

The medicament of the present invention is a composition which can be in any pharmaceutical form normally used for topical application, such as aqueous dispersions, aqueous or oily suspensions, aqueous gels, anhydrous or lipophilic emulsions (lotions, creams or ointments) of liquid, semisolid or solid, obtained by dispersing a fatty phase in an aqueous phase (O/W) or conversely (W/H) in the presence or absence of emulsifier, or micro emulsions, micro capsules, micro particles or vesicular dispersions of ionic and/or nonionic.

Thus in a preferred embodiment, the medicament according to the present invention can be provided in any pharmaceutical dosage form normally used in the field of dermatology. Preferably, the medicament will be provided in the emulsion (lotions, creams, cream without emulsifier), suspension or gel form.

A composition herein described comprising Adapalene comprises the following ingredients by weight relative to the total weight of the composition:
- adapalene between 0, 1% and 0,3%
- at least one gelling agent 0.001% to 15% and more preferentially between 0.15% and 5%
- at least one chelating agent 0,01% to 1,5%
- at least one preservative agent 0,01% to 3%
- at least one wetting agent between 0.1% and 10%
- pH adjuster.
Among the chelating agents, it can be mentionned ethylene diamine tetraacetic acid (EDTA), diethylene triamine pentaacetic acid (DTPA), ethylene diaminetetraacetic acid di-(O-hydroxyphenyl acetic acid) (EDDHA) acid, hydroxy-2-ethylene diamine triacetic acid (HEDTA), ethyldiamine acid-di (O-methyl-p-hydroxy phenyl) acetic acid (EDDHMA) acid and ethylene diamine-di (5-carboxy-2-hydroxyphenyl) acetic acid (EDDCHA).
The preferred chelating agent is ethylenediaminetetraacetic acid (EDTA), sold in particular under the name Titriplex III®.

By way of example of gelling or suspending agents may come in the compositions of the invention include, the so-called carbomers sold under the generic name of Carbopol®; the carbomers sensitive to electrolytes, sold under the name ETD Carbopol 'Ultrez 10® by BF Goodrich, the polysaccharides of xanthan gum as Keltrol® sold by Kelco, guar gum, chitosans, cellulose and its derivatives such as hydroxyethylcellulose, in particular the product sold under the name Natrosol HHX 250 by Aqualon Company.
As preferred gelling agents it is cited the carbomers sold in particular under the names Carbopol 974P NF and Carbopol 980 NF.

Among the preservatives or preservative agents herein described mention is made of benzoic acid and its derivatives with benzyl alcohol, benzalkonium chloride, sodium benzoate, bronopol, chlorhexidine, chlorocresol and its derivatives, ethyl alcohol, phenethyl alcohol, phenoxyethanol, potassium sorbate, diazolidinylurea parabens such as propyl paraben or methyl paraben, alone or in mixtures.
By way of preferred preservatives include parabens, phenoxyethanol or benzalkonium chloride, alone or in combination.
Among the emollients that role is to moisturize the skin and facilitate the application of the formulation, preferably used are compounds such as glycerin, propylene glycol, dipropylene glycol, propylene glycol dipelargonate, lauroglycol, alone or in combination.

As an emollient preferred include propylene glycol.
The composition according to the description may comprise at least one ingredient selected from the following list:
- gelling agents such as the carbomers preferably Carbopol 974P NF and Carbopol 980 NF
- Chelating agent such as Disodium edentate (EDTA)
- Emollient such as propylene glycol
- wetting agents poloxamer and preferred poloxamer 124
- preservative agents such as parabens, phenoxyethanol or benzalkonium chloride alone or in combination.

In a specific aspect, the composition comprises the following ingredients by range of weight relative to the total weight of the composition:

| | |
|---|---|
| Adapalene | 0.1% to 0.3% |
| Carbomer | 0.15 to 2% |
| Disodium edentate | 0.05% to 0.5% |
| Methyl paraben | 0.1% to 0.5% |
| Poloxamer | 0.1% to 0.5% |
| Propylene glycol | 4.0 % |
| Sodium hydroxide amount required to obtain pH 5.0 +/- 0.3 and | |
| Purified water | q.s. 100% |

Advantageously, the medicament according to the present invention corresponds to the aqueous gel sold by Galderma and comprising Adapalene at 0.3%, as presented in Example 1.
Furthermore, the composition as described above can comprise all the constituents normally present in the type of application herein described.

The medicament according to the present description can comprise a large variety of additional components; in particular, they can be absorbents, abrasives, antiacne agents, antifoaming agents, antimicrobial agents, antioxidants, binders, biological additives, buffers, chelating agents, colorants, cosmetic astringents, cosmetic biocides, external analgesics, film-forming agents, fragrance components, opacifying agents, plasticizers, preservatives, other depigmenting agents, emollients, skin-protecting agents, solvents, solubilizing agents, surfactants, agents which absorb ultraviolet light, sunscreens, viscosity-increasing agents (aqueous or non-aqueous), humectants and sequestering agents.

These additives may be present in the composition in an amount of 0.001 % to 20% by weight relative to the total weight of the composition. In the context of the present invention, the efficacy and safety of Adapalene Gel 0.3% in the treatment of atrophic acne scars are assessed by different technologies known by the skilled in the art. In particular, the efficacy is assessed clinically (including 2-D and 3-D photographs) and at molecular and cellular levels (IHC and transcriptomic).

As photodamage lesions and atrophic acne scars are both linked to a dermal matrix loss, there is a potential beneficial effect of Adapalene Gel 0.3% in the treatment of atrophic acne scars. Indeed by stimulating dermal fibroblasts to produce more procollagen, Adapalene Gel 0.3% applications during a 6-month period may result in improved clinical appearance of the atrophic acne scars.

The present invention will be now illustrated by the following examples.

### EXAMPLES:

### Example 1: gel with 0.3% of adapalene

| | |
|---|---|
| Adapalene | 0.3% |
| Carbomer | 1.1% |
| Disodium edentate | 0.1% |
| Methyl paraben | 0.2% |
| Poloxamer | 0.2% |
| Propylene glycol | 4.0 % |
| Sodium hydroxide amount required to obtain pH 5.0 +/- 0.3 and | |
| Purified water | q.s. 100% |

### Example 2: Efficacy and safety assessment of Adapalene Gel 0.3% in the treatment of atrophic acne scars.

This example provides a demonstration that a composition with 0.3% Adapalene provides efficient treatment of atrophic acne scars.

### 1. CLINICAL STUDY PROTOCOL

In this study, the efficacy are assessed clinically (including 2-D and 3-D photographs) and at molecular and cellular levels (IHC and transcriptomic).

For the study, a total of 20 subjects are enrolled in one site in the USA who are male or female subjects of any race aged 18 years or older with facial atrophic acne scars and meeting the following inclusion/exclusion criteria (among other criteria):
- Subjects with moderate to severe facial atrophic acne scars (grade 3 or 4)

| **Grade** | **Level of disease** | **Characteristics** | **Examples of scars** |
|---|---|---|---|
| 3 | Moderate disease | Moderate atrophic scarring that is obvious at social distances of 50 cm or greater and is not covered easily by makeup or the normal shadow of shaved beard hair in males, but is still able to be flattened by manual stretching of the skin | More significant rolling, shallow « boxcar» |
| 4 | Severe disease | Severe atrophic scarring that is obvious at social distances of 50 cm or greater and is not covered easily by makeup or the normal shadow of shaved beard hair in males and is not able to be flattened by manual stretching of the skin | Punched out atrophic (deep « boxcar»), « ice pick », bridges and tunnels, gross atrophy, dystrophic scars |
| Goodman GJ, Baron JA. Postacne scarring - a qualitative global scarring grading system. Dermatol Surg. 2006;32:1458-1466. | | | |

- Subjects with at least 2 similar facial boxcar acne scars eligible for biopsy,
- Subjects with 3 target boxcar or rolling acne scars as well as a target area containing at least 3 other boxcar or rolling acne scars eligible for photography,

| | |
|---|---|
| **Rolling scars** | Wider than 4 to 5mm. Abnormal fibrous anchoring of the dermis to the subcutis leads to superficial shadowing and a rolling or undulating appearance to the overlying skin. |
| **Boxcar scars** | Round to oval depressions with sharply demarcated vertical edges similar to varicella scars. Wider at the surface than ice pick scars and do not taper to a point at the base. They may be shallow (0.1 to 0.5mm) or deep (>0.5mm) and are most often 1.5 to 4.0 mm in diameter. |

### 1.1. OVERALL STUDY DESIGN

This study is conducted as mono-center, open-label study. This study involves subjects of any race, aged 18 years inclusive or older with facial atrophic acne scars and meeting specific inclusion/exclusion criteria.

Subjects are screened 2 weeks before Baseline visit to determine HIV, HBV & HCV serology. If HIV, HBV & HCV serology negative, subjects are enrolled at Baseline and treated for 24 weeks with Adapalene Gel 0.3% once daily application during the first 4 weeks and then twice daily application.

There are 7 on-site study visits: Screening/Week -2 (±5 days), Baseline, Week 2 (±2 days), Week 4 (±3 days), Week 8 (± 3 days), Week 16 (± 5 days) and Week 24 (± 5 days).Furthermore, two phone calls to reach subjects will be made at Weeks 12 (± 5 days)and 20 (± 5 days).

A 24-week period has been determined enough to obtain clinical effect and stimulate dermal fibroblasts to produce more procollagen in atrophic acne scars.

For each subject it is requested to:
- Apply Adapalene Gel 0.3% once daily in the evening on the whole face for the first 4 weeks,
   And then
- Apply Adapalene Gel 0.3% twice daily (in the morning and in the evening) on the whole face for the following 20 weeks,

2-D and 3-D photographs are performed during this study with 3 different systems and are analyzed to correlate findings with clinical assessments (3-D systems will permit to assess acne scar volume, surface and maximal depth of the 3 target atrophic acne scars (boxcar or rolling type) but also roughness of the target area containing at least 3 atrophic acne scares (boxcar or rolling type) when 2-D system will permit to evaluate overall facial skin roughness).

Biopsy size was validated beforehand in order to reduce the size of the punch as much as possible. A size of 3 mm in diameter is considered enough to perform all the tests required by this protocol and especially to recover RNA in good quantity and quality.

### 1.2. STUDY FLOW CHART

| **PROCEDURES** | **STUDY VISITS** | | | | | | | | | |
|---|---|---|---|---|---|---|---|---|---|---|
| | **Screening Week-2 (±5 days)** | **Baseline** | **Week 2 (±2 days)** | **Week 4 (±3 days)** | **Week 8 (±3 days)** | **Week 12 (±3 days) Phone call** | **Week 16 (±5 days)** | **Week 20 (±5 days) Phone call** | **Week 24 (±5 days)** | **Final a proced ures** |
| Demographics/Medica 1 history | | X | | | | | | | | |
| Concomitant treatment/Procedure | | X | X | X | X | [X]**^{d}** | X | [X]^{d} | X | |
| Global atrophic acne scar severity | | X | | | | | | | | X |
| Global assessment of improvement | | | X | X | X | | X | | X | |
| Local tolerability | | X | X | X | X | | X | | X | |
| Adverse events**^{e}** | X | X | X | X | X | [X]^{d} | X | [X]^{d} | X | |
| Subject satisfaction questionnaire | | | | | | | | | | X |
| Subject quality of life questionnaire | | X | | | | | | | | X |
| Blood sample | X | | | | | | | | | |
| Biopsy | | X | | | | | | | | X |
| Photograph procedures | | X | | | X | | X | | X | |
| Skin replica**^{f}** | | X | | | | | | | | |

| | | | | | | | | | | |
|---|---|---|---|---|---|---|---|---|---|---|
| d. Adverse event onsets after subject signature of the informed consent form should be recorded on the AE form of the CRF. | | | | | | | | | | |

### 1.2.1 Method of treatment assignment

In this open-label study no randomization list is issued by the statistician.

For treatment allocation, each subject who fulfils all inclusion/exclusion criteria receive the Baseline treatment box (Week 0) allocated in the chronological order of his/her entry in the study, and no number should be omitted or skipped.

### 1.2.2. Instructions for use and administration

The study treatment dispenser gives each subject verbal and written instructions on how to use the study treatment at on-site visit. Subject kit is dispensed according to the chronological order of enrollment of subjects into the study.

Subjects apply Adapalene Gel 0.3% on the whole face once daily in the evening for the first 4 weeks and then twice daily (in the morning and in the evening) for the following 20 weeks. The study treatment is used for 24 weeks in total.

The treatment administration is further described below.

| | **Adapalene Gel 0.3%** |
|---|---|
| Concentration | 0.3% |
| Dose Regimen | Once daily in the evening for first 4 weeks |
| | Then twice daily in the morning and in the evening for the following 20 weeks |
| Period of Administration | 24 weeks |
| Route of Administration | Topical |

### 2. EFFICACY AND SAFETY ASSESSMENT

Clinical evaluations are performed by the same Investigator throughout the study.

If it is not possible to use the same Investigator to follow the subject, then evaluations should overlap for at least one visit in order to examine the subject together and discuss findings.

### 2.1. EFFICACY ASSESSMENT

### 2.1.1. Global atrophic acne scar severity

Global atrophic acne scar severity score is automatically calculated by statistician according to ECCA grading scale.²⁹

Semi-quantitative score for each type of atrophic scar²⁹ (V-shaped [diameter of less than 2mm and puncti form], U-shaped [diameter of 2-4mm with sheer edges], M-shaped [diameter of more than 4mm, superficial and with irregular surface] and Superficial elastolysis) is determined by Investigator at Baseline and Week 24/Early termination visits as follows:

| **Semi-quantitative score** | |
|---|---|
| **0** | No scar |
| **1** | A few scars (less than 5) |
| **2** | Limited number of scars (between 5 and 20) |
| **3** | Many scars (more than 20) |

### 2.1.2. Global assessment of improvement

Facial skin texture, atrophic acne scars, and overall satisfaction is graded by the Investigator at each post-Baseline on-site visit as follows:

| **Facial skin texture** | |
|---|---|
| **0** | Worse |
| **1** | No change |
| **2** | 1-25% = slight improvement |
| **3** | 26-50% = moderate improvement |
| **4** | 51-75% = marked improvement |
| **5** | 75-90% = almost complete improvement |
| **6** | 90-100% = complete improvement |

| **Facial atrophic acne scars** | |
|---|---|
| **0** | Worse |
| **1** | No change |
| **2** | 1-25% = slight improvement |
| **3** | 26-50% = moderate improvement |
| **4** | 51-75% = marked improvement |
| **5** | 75-90% = almost complete improvement |
| **6** | 90-100% = complete improvement |

| **Overall satisfaction** | |
|---|---|
| **0** | Worse |
| **1** | No change |
| **2** | 1-25% = slight improvement |
| **3** | 26-50% = moderate improvement |
| **4** | 51-75% = marked improvement |
| **5** | 75-90% = almost complete improvement |
| **6** | 90-100% = complete improvement |

### 2.1.3. Photographs and replicas

2-D and 3-D photographs are performed during this study with 3 different systems and are analyzed to correlate findings with clinical assessments (3-D systems will permit to assess acne scar volume, surface and maximal depth of the 3 target atrophic acne scars (boxcar or rolling type) but also roughness of the target area containing at least 3 atrophic acne scares (boxcar or rolling type) when 2-D system will permit to evaluate overall facial skin roughness).

### 2-D photographs:

Randomly for each subject, five (5) areas for each cheek are photographed at Baseline, Week 8, Week 16 and Week 24/Early termination using a 2-D capture photograph system:
- Visioscan®

Photos with this specific digital 2-D capture photograph system are conducted at the investigational site. These photographs are performed according to a specific training and procedures which are provided to the Investigative site.

All 2-D images are analyzed digitally with computer-assisted tracking system to evaluate changes over time.

### 3-D photographs:

Three (3) target boxcar or rolling acne scars as well as a target area containing at least 3 other boxcar or rolling acne scars are photographed at Baseline, Week 8, Week 16 and Week 24/Early termination using two different 3-D capture photograph systems:
- 3D Lifeviz™ Micro
- Primos™ pico

Photos with these 2 different specific digital 3-D capture photograph systems are conducted at the investigational site. These photographs are performed according to a specific training and procedures which are provided to the Investigative site.
All 3-D images are analyzed digitally with computer-assisted tracking system to evaluate changes over time.

### Replicas:

At Baseline visit, for the 5 first enrolled subjects, skin replicas of the photographed areas are performed by using specific silicon (Silflo®) and by following provided procedure. These atrophic acne scar replicas permit to confirm 3-D images measurements.

### 2.1.4. Biopsies

### 2.1.4.1. Analysis

Explants obtained form biopsies are used exclusively in the framework of this study protocol.

Large-scale differential analysis of the transcriptome using micro-arrays (Affymetrix®) are performed on the 10 last subject explants. In order to validate differential profile, RT-PCR analysis are performed to confirm changes in selected genes expression. Bioinformatics and biology analysis are also performed for gene expression profiling analysis.

### 2.1.4.2. Sampling method

A total of two biopsies are carried-out on the face of each subject (3 mm in diameter); the first one at Baseline and the second one at Week 24/Early termination. Biopsies are taken on two boxcar scars similar in terms of severity. The second selected facial boxcar scar are described in the CRF and localized on a transparent-template permitting re-localization of it at Week 24/Early termination visit. Two explants from the first 10 subjects are necessary for IHC analysis. Two explants from the last 10 subjects are necessary for large-scale gene expression profiling.

### 2.2. SAFETY ASSESSMENT

### 2.2.1. Tolerability assessment

Erythema, scaling, dryness, and stinging/burning are graded at Baseline and at each post-Baseline on-site visit as follows:

| **Erythema** - abnormal redness of the skin. | | |
|---|---|---|
| **None** | **0** | No erythema |
| **Mild** | **1** | Slight pinkness present |
| **Moderate** | **2** | Definite redness, easily recognized |
| **Severe** | **3** | Intense redness |

| **Scaling** - abnormal shedding of the stratum corneum. | | |
|---|---|---|
| **None** | **0** | No scaling |
| **Mild** | **1** | Barely perceptible shedding, noticeable only on light scratching or rubbing |
| **Moderate** | **2** | Obvious but not profuse shedding |
| **Severe** | **3** | Heavy scale production |

| **Dryness** - brittle and/or tight sensation. | | |
|---|---|---|
| **None** | **0** | No dryness |
| **Mild** | **1** | Slight but definite roughness |
| **Moderate** | **2** | Moderate roughness |
| **Severe** | **3** | Marked roughness |

| **Stinging/burning** - prickling pain sensation. | | |
|---|---|---|
| **None** | **0** | No stinging/burning |
| **Mild** | **1** | Slight warm, tingling/stinging sensation; not really bothersome |
| **Moderate** | **2** | Definite warm, tingling/stinging sensation that is somewhat bothersome |
| **Severe** | **3** | Hot, tingling/stinging sensation that has caused definite discomfort |

Erythema, scaling, and dryness are evaluated, by the Investigator. Stinging/burning will be recorded by the Investigator after discussion with the subject.

### 2.2.2. Adverse events (AEs)

Adverse events are recorded during each follow up visit. All clinical medical events, whether observed by the Investigator or reported by the subject and whether or not thought to be study treatment-related or study procedure-related are considered adverse events and recorded.

### 2.3. SUBJECT RELATED OUTCOMES

### 2.3.1. Subject satisfaction questionnaire

At Week 24/Early termination, subjects complete a satisfaction questionnaire regarding the treatment used in this study.

### 2.3.2. DLQI questionnaire

DLQI questionnaire is collected at Baseline and Week 24/Early termination. The Investigator provides the subject with the DLQI form and instructs the subject to read and answers all ten quality of life questions.

This questionnaire measures the dermatology-related limitations of functional ability and the frequency, severity and impact of disease symptoms on subject' lives and disease-related quality of life.

The six areas addressed in the questionnaire are: symptoms and feelings; daily activities; leisure; work/school; personal relationships; and treatment.

### 3. STATISTICAL METHODS

### 3.1. STATISTICAL AND ANALYTICAL PLANS

The main purpose of this exploratory study is to demonstrate the effect of Adapalene Gel 0.3% in atrophic acne scars by using clinical signs assessments, 2-D and 3-D photographs analysis and skin molecular and cellular analysis.

### 3.1.1. Variables to be statistically analyzed

**Efficacy variable,** at each evaluation time
- Global atrophic acne scar severity score
- Investigator global assessment of improvement for facial skin texture, atrophy and overall satisfaction
- Profile of the selected acne scars and area over time by photography analysis
- Gene expression study using IHC and large scale gene profiling expression at Baseline and Week 24 /Early termination.

**Safety variables,** at each evaluation time
- Local tolerability parameters
- Incidence of Adverse Event(s)

### Subject related outcomes,

- Subject satisfaction questionnaire
- Subject quality of life questionnaire total score

### 3.1.2. Populations analyzed, evaluability and limitation / Evaluation of bias

The following populations are analyzed:

### 1. The intent-to-treat population (ITT)

This population consists of the entire population enrolled.

### 2. The safety population (APT)

This population consists of the Intent-to-Treat population, after exclusion of subjects who never applied study treatment with certainty based on CRF data and monitoring reports.

### 3. Missing values

No missing values are replaced (observed data).

### 3.1.3. Statistical analyses

No inferential statistics are performed. All variables are only descriptively summarized on ITT or APT population.

The intent-to-treat (ITT) population consists of the entire population enrolled. All patient treated (APT) population consists of the intent-to-treat population, after exclusion of subjects who never received any study treatment.

All variables assessed are presented at each evaluation time, in terms of raw values. Additionally, the change from Baseline in global atrophic acne scar severity score is analyzed.

For local tolerance variables, the worst-score post-Baseline is also evaluated to maximize the side effect.

The subject disposition, demographics and adverse event(s) are also descriptively summarized.

Regarding IHC in the 10 first subject-biopsied skin explants the following data are obtained from the IHC analysis at Investigative site:
Regarding gene expression in the 10 last subject-biopsied skin explants the following data are obtained from the arrays:
- Gene ID,
- Probe ID,
- Fold expression change in relation to a baseline expression,
- Known or predicted function,
- Member of pathway, where available,
- Gene ontology prediction of function, where available.

In each of the 10 last subjects, large scale gene expression analysis using micro-arrays (Affymetrix®) are used in order to identify profile at Baseline and at Week 24/Early termination. Extraction of mRNA as well as the steps involved in hybridisation of Affymetrix® U133A 2.0 Gene-chips array and normalisation of the raw data are performed. Data obtained are analyzed by a statistician and bioinformaticist to assess the impact of the study treatment. Functional annotation of differentially expressed genes are conducted using both freely (AMIGO) and software (Ingenuity pathway). Differentially expressed genes of specific signaling pathways are determined using available databases Biocarta and Genmapp. When appropriate, quantitative RT-PCR on an ABI 7000 are used to confirm changes in the levels of expression of key genes.

## Claims

1. Composition comprising adapalene or its salts in a quantity of 0.3% of weight relative to the total weight of the composition for use in a method for treatment by therapy of at least one acne scar, wherein adapalene or its salts is used as unique active ingredient.

2. Use of a composition comprising adapalene or its salts in a quantity of 0.3% of weight relative to the total weight of the composition for non-therapeutic treatment of acne scar, wherein adapalene or its salts is used as the unique active ingredient.

3. Composition comprising adapalene or its salts for use according claim 1 or use of a composition comprising adapalene or its salts according to claim 2, wherein an effective amount of adapalene or its salts is in a composition in the form of an aqueous gel.

4. Composition comprising adapalene or its salts for use according to anyone of claims 1 or 3 or use of a composition comprising adapalene or its salts according to claim 2 or 3, wherein acne scar is an atrophic scar and wherein the composition is a gel which further contains as percentage by weight with respect to the total weight:
| | |
|---|---|
| Carbomer | 1.1% |
| Disodium edentate | 0.1% |
| Methyl paraben | 0.2% |
| Poloxamer | 0.2% |
| Propylene glycol | 4.0 % |
| Sodium hydroxide amount required to obtain pH 5.0 +/- 0.3 and Purified water | q.s. 100%. |

5. Composition comprising adapalene or its salts for use or use of a composition comprising adapalene or its salts according to claim 4, wherein acne scar is selected from boxcar and rolling scars.

6. Composition comprising adapalene or its salts for use according to anyone of claims 1 or 3-5, or use of a composition comprising adapalene or its salts according to anyone of claims 2-5, wherein the effective amount of adapalene is topically administrated.

7. Composition comprising adapalene or its salts for use according to anyone of claims 1 or 3-6, or use of a composition comprising adapalene or its salts according to anyone of claims 2-6, wherein the effective amount of adapalene is applied onto scarred skin.

8. Use of Adapalene or its salts at 0.3% for the preparation of a medicament which is intended for the treatment of acne scars, wherein adapalene or its salts is used as the unique active ingredient.

9. Use according to claim 8, **characterized in that** the medicament is in the form of a composition suitable for topical application.

10. Use according to any one of claims 8 or 9, **characterized in that** the medicament is in the form of a gel, emulsion or lotion.

11. Use according to any one of claims 8 to 10, **characterized in that** the medicament is in the form of a composition comprising aqueous gel having the following composition, as percentage by weight with respect to the total weight:
| | |
|---|---|
| Adapalene | 0.3% |
| Carbomer | 1.1% |
| Disodium edentate | 0.1% |
| Methyl paraben | 0.2% |
| Poloxamer | 0.2% |
| Propylene glycol | 4.0 % |
| Sodium hydroxide amount required to obtain pH 5.0 +/- 0.3 and Purified water | q.s. 100% |

## Patentansprüche

1. Eine Zusammensetzung umfassend Adapalen oder seine Salze in einer Menge von 0,3% des Gewichts relativ zu dem Gesamtgewicht der Zusammensetzung zur Verwendung in einem Verfahren zur Behandlung durch Therapie mindestens einer Aknenarbe, wobei Adapalen oder seine Salze als einziger Wirkstoff verwendet wird.

2. Verwendung einer Zusammensetzung umfassend Adapalen oder seiner Salze in einer Menge von 0,3% des Gewichts relativ zu dem Gesamtgewicht der Zusammensetzung für eine nicht-therapeutische Behandlung einer Aknenarbe, wobei Adapalen oder seine Salze als der einzige Wirkstoff verwendet wird.

3. Zusammensetzung umfassend Adapalen oder seine Salze zur Verwendung nach Anspruch 1 oder Verwendung einer Zusammensetzung umfassend Adapalen oder seiner Salze nach Anspruch 2, wobei eine effektive Menge Adapalen oder seiner Salze in einer Zusammensetzung ist in der Form eines wässrigen Gels.

4. Zusammensetzung umfassend Adapalen oder seiner Salze zur Verwendung nach einem der Ansprüche 1 oder 3 oder Verwendung einer Zusammensetzung umfassend Adapalen oder seiner Salze nach Anspruch 2 oder 3, wobei die Aknenarbe eine atrophische Narbe ist, und wobei die Zusammensetzung ein Gel ist, welches weiter enthält als Gewichtsprozent im Hinblick auf das Gesamtgewicht:
| | |
|---|---|
| Carbomer | 1,1% |
| Dinatriumedetat | 0,1% |
| Methylparaben | 0,2% |
| Poloxamer | 0,2% |
| Propylenglycol | 4,0% |
| Eine Menge Natriumhydroxid, benötigt, um einen pH von 5,0 +/- 0,3 zu erhalten und gereinigtes Wasser | ad 100%. |

5. Zusammensetzung umfassend Adapalen oder seine Salze zur Verwendung oder Verwendung einer Zusammensetzung umfassend Adapalen oder seiner Salze nach Anspruch 4, wobei die Aknenarbe ausgewählt ist aus Boxcar-Narben und rollenden Narben.

6. Zusammensetzung umfassend Adapalen oder seine Salze zur Verwendung nach einem der Ansprüche 1 oder 3-5, oder Verwendung einer Zusammensetzung umfassend Adapalen oder seiner Salze nach einem der Ansprüche 2-5, wobei die effektive Menge an Adapalen topisch verabreicht wird.

7. Zusammensetzung umfassend Adapalen oder seiner Salze zur Verwendung nach einem der Ansprüche 1 oder 3-6, oder Verwendung einer Zusammensetzung umfassend Adapalen oder seiner Salze nach einem der Ansprüche 2-6, wobei die effektive Menge an Adapalen auf vernarbte Haut aufgetragen wird.

8. Verwendung von Adapalen oder seiner Salze von 0,3% zur Herstellung eines Medikaments, welches für die Behandlung von Aknenarben beabsichtigt ist, wobei Adapalen oder seine Salze als einziger Wirkstoff verwendet wird.

9. Verwendung nach Anspruch 8 **dadurch gekennzeichnet, dass** das Medikament in der Form einer Zusammensetzung geeignet für die topische Verabreichung vorliegt.

10. Verwendung nach einem der Ansprüche 8 oder 9 **dadurch gekennzeichnet, dass** das Medikament in der Form eines Gels, Emulsion oder Lotion vorliegt.

11. Verwendung nach einem der Ansprüche 8 bis 10 **dadurch gekennzeichnet, dass** das Medikament in der Form einer Zusammensetzung vorliegt umfassend ein wässriges Gel mit der folgenden Zusammensetzung als Gewichtsprozent im Hinblick auf das Gesamtgewicht:
| | |
|---|---|
| Adapalen | 0,3% |
| Carbomer | 1,1% |
| Dinatriumedetat | 0,1% |
| Methylparaben | 0,2% |
| Poloxamer | 0,2% |
| Propylenglycol | 4,0% |
| Eine Menge Natriumhydroxid, benötigt, um einen pH von 5,0 +/- 0,3 zu erhalten und gereinigtes Wasser | ad 100%. |

## Revendications

1. Composition comprenant de l'adapalène ou ses sels en une quantité de 0,3% en poids par rapport au poids total de la composition pour son utilisation dans une méthode de traitement par thérapie d'au moins une cicatrice d'acné, dans laquelle l'adapalène ou ses sels est utilisé comme principe actif unique.

2. Utilisation d'une composition comprenant de l'adapalène ou ses sels en une quantité de 0,3% en poids par rapport au poids total de la composition pour un traitement non-thérapeutique de cicatrice d'acné, dans laquelle l'adapalène ou ses sels est utilisé comme principe actif unique.

3. Composition comprenant de l'adapalène ou ses sels pour son utilisation selon la revendication 1 ou utilisation d'une composition comprenant de l'adapalène ou ses sels selon la revendication 2, dans laquelle une quantité efficace d'adapalène ou ses sels est dans une composition sous la forme d'un gel aqueux.

4. Composition comprenant de l'adapalène ou ses sels pour son utilisation selon l'une quelconque des revendications 1 ou 3 ou utilisation d'une composition comprenant de l'adapalène ou ses sels selon la revendication 2 ou 3, dans laquelle la cicatrice d'acné est une cicatrice atrophique et dans laquelle la composition est un gel qui contient en outre en pourcentage en poids par rapport au poids total :
| | |
|---|---|
| Carbomère | 1,1% |
| Édentate disodique | 0,1% |
| Méthyle parabène | 0,2% |
| Poloxamère | 0,2% |
| Propylène glycol | 4,0% |
| Quantité d'hydroxyde de sodium requise pour obtenir pH 5,0 +/- 0,3 et Eau purifiée | qsp 100% |

5. Composition comprenant de l'adapalène ou ses sels pour son utilisation ou utilisation d'une composition comprenant de l'adapalène ou ses sels selon la revendication 4, dans laquelle la cicatrice d'acné est sélectionnée parmi les cicatrices à bords prononcés (« boxcar ») et les cicatrices d'aspect roulant (« rolling scar »).

6. Composition comprenant de l'adapalène ou ses sels pour son utilisation selon l'une quelconque des revendications 1 ou 3-5, ou utilisation d'une composition comprenant de l'adapalène ou ses sels selon l'une quelconque des revendications 2-5, dans laquelle la quantité efficace d'adapalène est administrée par voie topique.

7. Composition comprenant de l'adapalène ou ses sels pour son utilisation selon l'une quelconque des revendications 1 ou 3-6, ou utilisation d'une composition comprenant de l'adapalène ou ses sels selon l'une quelconque des revendications 2-6, dans laquelle la quantité efficace d'adapalène est administrée sur la peau cicatrisée.

8. Utilisation de l'Adapalène ou de ses sels à 0,3% pour la préparation d'un médicament qui est destiné au traitement des cicatrices d'acné, dans laquelle l'adapalène ou ses sels est utilisé comme principe actif unique.

9. Utilisation selon la revendication 8, **caractérisée en ce que** le médicament est sous la forme d'une composition appropriée pour une application topique.

10. Utilisation selon l'une quelconque des revendications 8 ou 9, **caractérisée en ce que** le médicament se présente sous la forme d'un gel, d'une émulsion ou d'une lotion.

11. Utilisation selon l'une quelconque des revendications 8 ou 9, **caractérisée en ce que** le médicament est sous la forme d'une composition comprenant un gel aqueux ayant la composition suivante, en pourcentage en poids par rapport au poids total :
| | |
|---|---|
| Adapalène | 0,3% |
| Carbomère | 1,1% |
| Édentate disodique | 0,1% |
| Méthyle parabène | 0,2% |
| Poloxamère | 0,2% |
| Propylène glycol | 4,0% |
| Quantité d'hydroxyde de sodium requise pour obtenir pH 5,0 +/- 0,3 et Eau purifiée | qsp 100% |
